# EUROPEAN PATENT SPECIFICATION

(11) **EP 4 454 687 B1**
(45) Date of publication and mention of the grant of the patent: **23.07.2025**
(21) Application number: 24170338.8
(22) Date of filing: 15.04.2024
(51) Int. Cl.: A61M 11/00, A61M 15/00

(54) **AEROSOL APPARATUS**
AEROSOLVORRICHTUNG
APPAREIL AEROSOL

(30) Priority: 27.04.2023 IT 202300008238
(43) Date of publication of application: 30.10.2024
(73) Proprietor: CA-MI Societa' a Responsabilita' Limitata, 43013 Langhirano (PR) (IT)
(72) Inventor: ATTOLINI, Mario, 43037 Lesignano de Bagni (Parma) (IT)
(74) Representative: Monelli, Alberto

(56) References cited:
- EP-A1- 3 838 004
- CN-U- 213 191 829
- CN-U- 218 652 669
- US-A1- 2016 361 506
- US-B2- 11 589 421

## Description

The present invention relates to an aerosol apparatus. Aerosol apparatuses are known comprising a main body intended to be held by a user's hand. The main body can have a weight of approximately 120 grams and provide housing for a nebulising means of a medical substance and power supply batteries. A mask extends from the main body for administering the nebulised product to the user.

Solutions are also known comprising a main body which houses the nebulising means and provided with an electrical power cable intended to connect to a smartphone. The main body can have a weight of approximately 30 grams and be provided with an elastic band through which it can be connected behind the head of a user (leaving the hands free).

Document CN 218 652 669 U describes a portable electronic nebulisation device for aerosol therapy comprising a first casing with the nebulisation means and a second casing with the power supply battery; the two casings are hooked but separable and electrically connected via an electrical cable both when the first and second casings are hooked and when they are unhooked. When the first and second enclosures are hooked, an additional winding system is required.

The object of the present invention is to propose an aerosol apparatus which allows to offer maximum flexibility of use by a user.

The defined technical task and the specified aims are substantially achieved by an aerosol apparatus comprising the technical characteristics set forth in one or more of the appended claims.

Further characteristics and advantages of the present invention will become more apparent from the following indicative and therefore non-limiting description of an apparatus as illustrated in the appended drawings, in which:

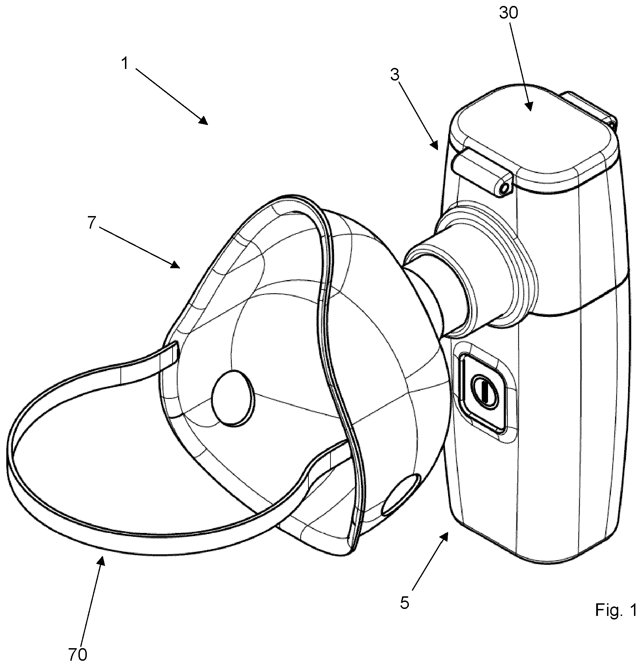

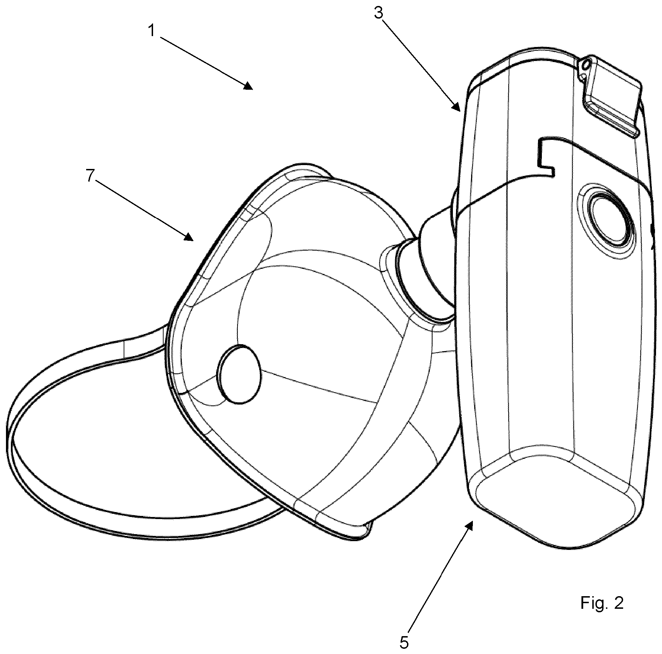

- figures 1 and 2 show two perspective views of the apparatus according to the present invention in a first configuration;

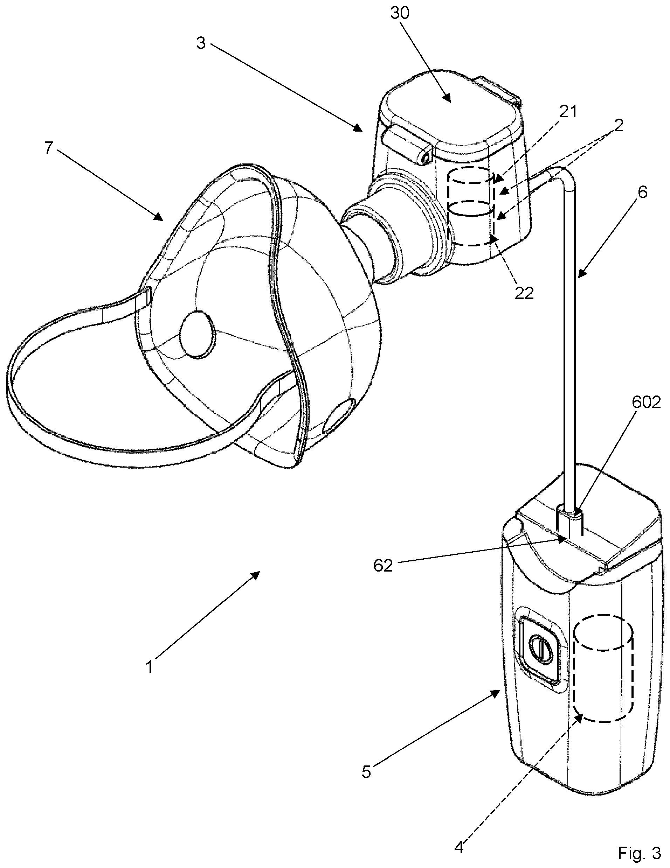

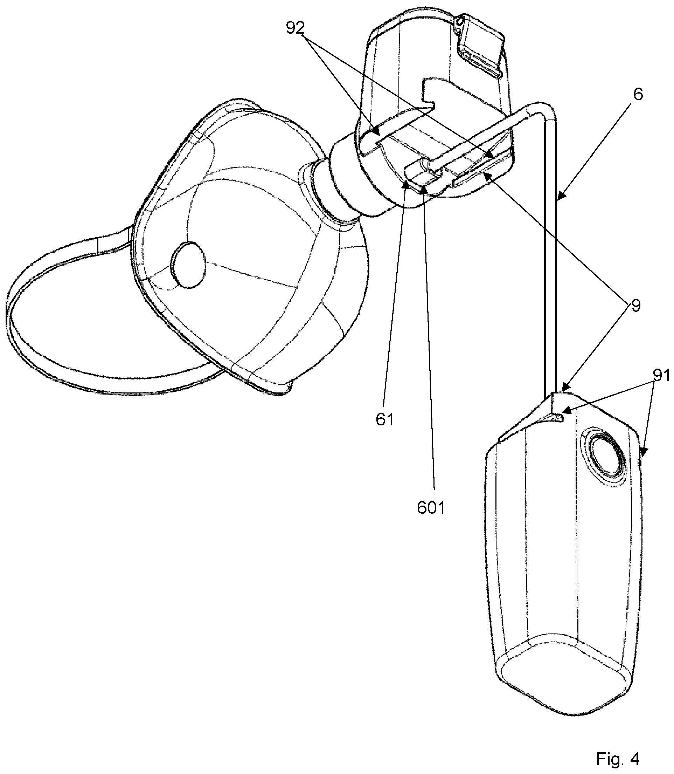

- figures 3 and 4 show two perspective views of the apparatus according to the present invention in a second configuration.

An aerosol apparatus is denoted in the appended figures by reference number 1. The aerosol apparatus 1 comprises a nebulising means 2 for nebulising a substance to be administered to a user. Such a substance is for example a medical-type substance or sterile saline solution.

The nebulising means 2 can comprise a reservoir 21 containing the substance to be nebulised (typically called an ampoule in technical jargon). The nebulising means 2 can comprise an actuator 22 which brings about the nebulisation of the substance.

Such an actuator 22 can be of various types. By way of non-limiting example, it could be a vibrating membrane or comprise a mechanism which generates ultrasound. It could possibly comprise a compressor.

Suitably, the apparatus 1 comprises a first casing 3 for housing at least said actuator 22. Suitably the first casing 3 comprises a cover 30 for access to the inside of the casing 3. Suitably, the cover 30 allows to access the reservoir 21.

The apparatus 1 also comprises an electrical energy storage means 4. The storage means 4 enables powering the apparatus 1, in particular the nebulising means 2. Suitably, the storage means 4 allows the (direct or indirect) electrical powering of said actuator 22 of the nebulising means 2. The electrical energy storage means 4 comprises/is an electric battery. The electrical energy storage means 4 is typically rechargeable (e.g., by means of connection to a power mains or other source of electrical energy). The storage means 4 can be sized to allow an operational autonomy comprised between 120 and 240 minutes.

The apparatus 1 comprises a second casing 5 for housing the electrical energy storage means 4.

Suitably, the first and second casings 3, 5 are movable between:
i) a first configuration wherein the first and second casings 3, 5 are solidly assembled to each other; and
ii) a second configuration wherein the second casing 5 is movable relative to the first casing 3.

For example, the first casing 3 and its content can have a weight comprised between 20 and 40 grams. For example, the second casing 5 and its content can have a weight comprised between 70 and 150 grams, preferably comprised between 80 and 100 grams.

The apparatus 1 suitably comprises a selector (e.g., a push button) placed on the second casing 5. An interface (e.g., a display or indicator light, etc.) can for example be obtained on the second casing 5.

Suitably, the second casing 5 comprises a support base which allows the second casing 5 (or more generally the apparatus 1) to stand without further support. Suitably, the second casing 5 has a preponderant extension direction in height. The first casing 3 has a smaller volume relative to the second casing 5.

In the first configuration, the first and second casings 3, 5 are adjacent. In particular, they are in direct contact. The first casing 3 is placed on a top of the second casing 5.

Suitably the electrical energy storage means 4 can electrically power the nebulising means 2 in both the first and second configurations.

The apparatus 1 comprises an electrical cable 6, which in the second configuration connects the first and second casings 3, 5, enabling the electrical powering of the nebulising means 2 by the electrical energy storage means 4.

The apparatus 1 comprises a first electrical connector 61 positioned along the first casing 3 and connected to the storage means 4.

The apparatus 1 comprises a second electrical connector 62 positioned along the second casing 5 and connected to the nebulising means 2. In the first configuration, the first and second connectors 61, 62 are reciprocally connected to enable the electrical powering of the nebulising means 2.

In the second configuration, the cable 6 connects the first and second connectors 61, 62.

The electrical cable 6 is removably connectable to the first and second connectors 61, 62.

In particular, the cable 6 comprises a first and second plug 601, 602 intended to connect one with the first connector 61 and one with the second connector 62.

Suitably, the first connector 61 is a USB connector. Suitably, the second connector 62 is also a USB connector.

Advantageously the first and/or the second plug 601, 602 are/is USB.

In the first configuration, the cable 6 is disconnected from the first and/or second connectors 61, 62.

Advantageously, the apparatus 1 comprises a mask 7 for the delivery of the nebulised substance by the nebulising means 2.

The mask 7 is associated with the first casing 3. The mask 7 comprises a connection system to the head of a user (e.g., an elastic band 70 which can be positioned behind the head).

The apparatus 1 comprises a removable connection means 9 which:
- is reciprocally coupled in the first configuration;
- is in part obtained in the first casing 3 and in part obtained in the second casing 5.

For example, one between the first and second casings 3, 5 can comprise two parallel flaps 91 and the other two parallel longitudinal grooves 92 in which the flaps 91 couple in the first configuration. Suitably, the flaps 91 are obtained in the second casing 5 and the grooves 92 are obtained in the first casing 3.

The removable connection means 9 can be quickly coupled. Typically, the connection means 9 can be quickly coupled without requiring tools.

The apparatus 1 can comprise a plug for drawing current from the mains electricity supply. The plug could, for example, be obtained on the second casing 5.

In a third configuration (not illustrated) of the apparatus 1, the first casing 3 is physically connectable to a tablet or smartphone or computer or other electronic device through electrical connection means (typically the cable 6) to enable electrical powering of the nebulising means 2. In such a case, the second casing 5 is unused.

The present invention achieves important advantages.

In fact, it allows multiple operating modes corresponding to the first, second or possibly third configuration. This allows to offer the operator maximum operational flexibility, being able to count on a light and versatile structure. In the first configuration, the user can grasp the first and second casings 3, 5 with one hand and perform the treatment. In the second configuration, the user can apply the mask 7 to the face (e.g., with the elastic band 70) and perform the treatment.

The invention thus conceived is susceptible to numerous modifications and variants as long as they fall under the scope of the claims. Furthermore, all the details may be replaced with other technically equivalent elements. All the materials used, as well as the dimensions, may in practice be any whatsoever according to needs.

The invention is defined by the following claims.

## Claims

1. An aerosol apparatus comprising:
- a nebulising means (2) for nebulising a substance to be administered to a user; the nebulising means (2) comprising a reservoir (21) containing the substance to be nebulised and an actuator (22) which brings about the nebulisation of the substance;
- a first casing (3) for housing at least said actuator (22);
- an electrical energy storage means (4) for powering said actuator (22) of the nebulising means (2);
- a second casing (5) for housing the electrical energy storage means (4); the first and second casings (3, 5) being movable between: i) a first configuration wherein the first and second casings (3, 5) are solidly assembled to each other; and
ii) a second configuration wherein the second casing (5) is movable relative to the first casing (3);
the electrical energy storage means (4) being able to electrically power the nebulising means (2) in both the first and second configurations;
the aerosol apparatus further comprising:
- a first electrical connector (61) positioned along the first casing (3) and connected to the storage means (4);
- a second electrical connector (62) positioned along the second casing (5) and electrically connected to the nebulising means (2); in the first configuration, the first and second connectors (61, 62) being reciprocally connected to enable the electrical powering of the nebulising means (2);
- an electrical cable (6), which in the second configuration connects the first and second casings (3, 5), enabling the electrical powering of the nebulising means (2) by the electrical energy storage means (4);
wherein said electrical cable (6), in the second configuration, connects the first and second connectors (61, 62);
**characterised in that** said electrical cable (6) is removably connectable to the first and second connectors (61, 62); said electrical cable (6), in the first configuration, being disconnected from the first and second connectors (61, 62).

2. The apparatus according to claim 1, **characterised in that** it comprises a mask (7) for the delivery of the nebulised substance by the nebulising means (2); said mask (7) being associated with the first casing (3).

3. The apparatus according to any one of the preceding claims, **characterised in that** it comprises removable connection means (9) which:
- in the first configuration are reciprocally coupled;
- are in part obtained in the first casing (3) and in the second casing (5).

4. The apparatus according to claim 3, **characterised in that** the removable connection means (9) can be quickly coupled without requiring tools.

5. The apparatus according to any one of the preceding claims, **characterised in that** it comprises a plug for drawing current from the mains electricity supply; said plug being obtained on the second casing (5).

6. The apparatus according to any one of the preceding claims, **characterised in that**, in a third configuration, the first casing (3) is physically connectable to a tablet or smartphone or computer through electrical connection means to enable electrical powering of the nebulising means (2).

7. The apparatus according to any one of the preceding claims, **characterised in that** said electrical energy storage means (4) is rechargeable.

## Patentansprüche

1. Aerosolvorrichtung umfassend:
- Zerstäubungsmittel (2) zum Vernebeln einer an einen Benutzer zu verabreichenden Substanz; wobei die Zerstäubungsmittel (2) ein Reservoir (21), das die zu vernebelnde Substanz enthält, und einen Aktuator (22) umfasst, der die Vernebelung der Substanz veranlasst;
- ein erstes Gehäuse (3) zum Aufnehmen mindestens des Aktuators (22);
- elektrische Energiespeichermittel (4) zum Versorgen des Aktuators (22) der Zerstäubungsmittel (2);
- ein zweites Gehäuse (5) zum Aufnehmen der elektrischen Energiespeichermittel (4); wobei das erste und das zweite Gehäuse (3, 5) beweglich sind zwischen: i) einer ersten Auslegung, in der das erste und das zweite Gehäuse (3, 5) fest miteinander zusammengebaut sind; und
ii) eine zweite Auslegung, in der das zweite Gehäuse (5) relativ zu dem ersten Gehäuse (3) beweglich ist;
wobei die elektrischen Energiespeichermittel (4) in der Lage sind, die Zerstäubungsmittel (2) sowohl in der ersten als auch in der zweiten Auslegung elektrisch zu versorgen;
wobei die Aerosolvorrichtung ferner Folgendes umfasst:
- einen ersten elektrischen Verbinder (61), der entlang des ersten Gehäuses (3) positioniert und mit den Speichermitteln (4) verbunden ist;
- einen zweiten elektrischen Verbinder (62), der entlang des zweiten Gehäuses (5) positioniert und elektrisch mit den Zerstäubungsmitteln (2) verbunden ist; wobei in der ersten Auslegung der erste und der zweite Verbinder (61, 62) wechselseitig verbunden sind, um die elektrische Versorgung der Zerstäubungsmittel (2) zu ermöglichen;
- ein elektrisches Kabel (6), das in der zweiten Auslegung das erste und zweite Gehäuse (3, 5) verbindet, wodurch die elektrische Versorgung der Zerstäubungsmittel (2) durch die elektrischen Energiespeichermittel (4) ermöglicht wird;
wobei das elektrische Kabel (6) in der zweiten Auslegung den ersten und zweiten Verbinder (61, 62) verbindet; **dadurch gekennzeichnet, dass** das elektrische Kabel (6) entfernbar mit dem ersten und zweiten Verbinder (61, 62) verbunden werden kann; wobei das elektrische Kabel (6) in der ersten Auslegung von dem ersten und zweiten Verbinder (61, 62) getrennt wird.

2. Vorrichtung nach Anspruch 1, **dadurch gekennzeichnet, dass** sie eine Maske (7) für die Abgabe der zerstäubten Substanz durch die Zerstäubungsmittel (2) umfasst; wobei die Maske (7) mit dem ersten Gehäuse (3) assoziiert ist.

3. Vorrichtung nach einem der vorhergehenden Ansprüche, **dadurch gekennzeichnet, dass** sie entfernbare Verbindungsmittel (9) umfasst, die:
- in der ersten Auslegung wechselseitig gekoppelt sind;
- teilweise im ersten Gehäuse (3) und im zweiten Gehäuse (5) erhalten werden.

4. Vorrichtung nach Anspruch 3, **dadurch gekennzeichnet, dass** die entfernbaren Verbindungsmittel (9) werkzeuglos schnell koppelbar sind.

5. Vorrichtung nach einem der vorhergehenden Ansprüche, **dadurch gekennzeichnet, dass** sie einen Stecker zum Entnehmen von Strom aus der Netzstromversorgung umfasst; wobei der Stecker an dem zweiten Gehäuse (5) erhalten wird.

6. Vorrichtung nach einem der vorhergehenden Ansprüche, **dadurch gekennzeichnet, dass** in einer dritten Auslegung das erste Gehäuse (3) physisch mit einem Tablet oder Smartphone oder Computer über elektrische Verbindungsmittel verbunden werden kann, um eine elektrische Versorgung der Zerstäubungsmittel (2) zu ermöglichen.

7. Vorrichtung nach einem der vorhergehenden Ansprüche, **dadurch gekennzeichnet, dass** die elektrischen Energiespeichermittel (4) wiederaufladbar sind.

## Revendications

1. Appareil aérosol comprenant:
- des moyens de nébulisation (2) pour nébuliser une substance à administrer à un utilisateur; les moyens de nébulisation (2) comprenant un réservoir (21) contenant la substance à nébuliser et un actionneur (22) qui provoque la nébulisation de la substance;
- un premier boîtier (3) pour loger au moins ledit actionneur (22);
- des moyens de stockage (4) d'énergie électrique pour alimenter ledit actionneur (22) des moyens de nébulisation (2);
- un second boîtier (5) pour loger les moyens de stockage (4) d'énergie électrique; les premier et second boîtiers (3, 5) étant mobiles entre: i) une première configuration dans laquelle les premier et second boîtiers (3, 5) sont solidement assemblés l'un à l'autre; et
ii) une deuxième configuration dans laquelle le second boîtier (5) est mobile par rapport au premier boîtier (3) ;
les moyens de stockage (4) d'énergie électrique pouvant alimenter électriquement les moyens de nébulisation (2) dans les première et deuxième configurations;
l'appareil aérosol comprenant en outre:
- un premier connecteur électrique (61) positionné le long du premier boîtier (3) et connecté aux moyens de stockage (4);
- un second connecteur électrique (62) positionné le long du second boîtier (5) et connecté électriquement aux moyens de nébulisation (2); dans la première configuration, les premier et second connecteurs (61, 62) étant réciproquement connectés pour permettre l'alimentation électrique des moyens de nébulisation (2) ;
- un câble électrique (6) qui, dans la deuxième configuration, connecte les premier et second boîtiers (3, 5), permettant l'alimentation électrique des moyens de nébulisation (2) par les moyens de stockage (4) d'énergie électrique;
dans lequel ledit câble électrique (6), dans la deuxième configuration, connecte les premier et second connecteurs (61, 62);
**caractérisé en ce que** ledit câble électrique (6) peut être connecté de manière amovible aux premier et second connecteurs (61, 62); ledit câble électrique (6), dans la première configuration, étant déconnecté des premier et second connecteurs (61, 62).

2. Appareil selon la revendication 1, **caractérisé en ce qu'**il comprend un masque (7) pour la distribution de la substance nébulisée par les moyens de nébulisation (2); ledit masque (7) étant associé au premier boîtier (3).

3. Appareil selon l'une quelconque des revendications précédentes, **caractérisé en ce qu'**il comprend des moyens de raccordement amovibles (9) qui:
- dans la première configuration sont accouplés réciproquement;
- sont en partie obtenus dans le premier boîtier (3) et dans le second boîtier (5).

4. Appareil selon la revendication 3, **caractérisé en ce que** les moyens de raccordement amovibles (9) peuvent être rapidement accouplés sans nécessiter d'outils.

5. Appareil selon l'une quelconque des revendications précédentes, **caractérisé en ce qu'**il comprend une prise pour tirer le courant du réseau électrique; ladite prise étant obtenue sur le second boîtier (5).

6. Appareil selon l'une quelconque des revendications précédentes, **caractérisé en ce que**, dans une troisième configuration, le premier boîtier (3) peut être physiquement connecté à une tablette ou un Smartphone ou un ordinateur par des moyens de connexion électrique pour permettre l'alimentation électrique des moyens de nébulisation (2).

7. Appareil selon l'une quelconque des revendications précédentes, **caractérisé en ce que** lesdits moyens de stockage (4) d'énergie électrique sont rechargeables.
